# EUROPEAN PATENT SPECIFICATION

(11) **EP 1 502 560 B1**
(45) Date of publication and mention of the grant of the patent: **19.07.2006**
(21) Application number: 04077622.1
(22) Date of filing: 10.05.2000
(51) Int. Cl.: A61F 2/16

(54) **Iris fixated intraocular lens**
Intraokulare Linse zur Befestigung an der Iris
Lentille intraoculaire fixée à l'iris

(30) Priority: 14.05.1999 US 312566; 21.01.2000 US 489069
(43) Date of publication of application: 02.02.2005
(62) Divisional of application: 00930534.3
(73) Proprietor: Portney, Valdemar, Tustin, CA 92680 (US)
(72) Inventor: Portney, Valdemar, Tustin, CA 92680 (US)
(74) Representative: Stainthorpe, Vanessa Juliet

(56) References cited:
- GB-A- 2 085 731
- US-A- 4 198 714
- US-A- 4 542 541
- US-A- 4 666 444
- US-A- 5 192 319

## Description

### FIELD OF THE INVENTION

The present invention relates generally to the field of ophthalmics, more particularly to ophthalmic devices, and still more particularly to ophthalmic devices known as intraocular lenses (IOLs).

### BACKGROUND DISCUSSION

At the onset it may be helpful to the understanding of the present invention to define the term "phakic" as relates to human eyes. The term "phakic" is applied to an eye in which the natural ocular lens is still present. This is in contrast to an aphakic eye from which the natural ocular lens has for any reason been removed. A phakic eye is considered a dynamic or active eye because the living natural lens is subject to change over time, while an aphakic eye is considered a static eye because the natural lens has been removed.

Vision in an eye is enabled by light from a viewed image being refracted to the retina by the cornea and the natural lens (and/or any implanted intraocular lens) located posterior of the cornea.

One relatively common ocular problem is impaired or complete loss of vision due to the natural ocular lens becoming cloudy or opaque, a condition known as cataract. The formation of cataracts is typically associated with natural bodily ageing, and most individuals over the age of about 60 years suffer from cataracts at least to some extent.

Cataracts cannot currently be cured, reversed, or even significantly arrested. Accordingly, the corrective action involves surgically removing the natural lens when impaired, the results being that a phakic eye becomes an aphakic eye.

After a defective natural lens has been surgically removed, the current vision-restoring practice (since about the 1940's) is to implant in the aphakic eye an artificial refractive lens called an intraocular lens (IOL) having an optic and optic fixation means. Previously, thick, heavy, high diopter spectacles were prescribed for aphakic eyes. Such spectacles however were and still are generally disliked by most patients for their weight and appearance:

Implantable IOLs were initially constructed from rigid polymethyl methacrylate (PMMA), a hard, biocompatable plastic material. More recently, IOLs have been constructed from a soft, elastically deformable, silicone or acrylate material that enables insertion of the IOLs through small ocular incisions.

In addition to the implanting of IOLs in aphakic eyes to restore vision after removal of the natural lens, considerable interest has recently arisen in implanting IOLs in phakic eyes to correct myopia, hyperopia, presbyopia or astigmatism problems associated with non-cataract natural lenses. This implanting of corrective IOLs in phakic eyes is an often-attractive alternative to the wearing of corrective spectacles or contact lenses, which limit certain activities and even certain professions, or having performed such surgical procedures on the cornea as radial keratomy (RK) or photo-radial keratectomy (PRK), which may not be desired by many individuals for various reasons. The implanting of refractive IOLs in phakic eyes to correct vision problems is considered to constitute one of the remaining frontiers of vision correction.

In an aphakic eye, a replacement IOL is now typically implanted in the posterior chamber of the eye from which the natural lens has been removed. In contrast, a corrective IOL for a phakic eye is most desirably implanted in the anterior chamber of the eye, forwardly of the intact natural lens in the posterior chamber of the eye. The former is called a posterior chamber IOL and the latter is called an anterior chamber IOL, and there are significant construction differences between the two types of IOLs.

With regard to anterior chamber lOLs, there has been renewed interest in IOLs constructed for fixation to the iris (some of the earliest IOLs were iris fixated, anterior chamber IOLs). By fixing the optic supporting structure to the iris itself, contact with the sensitive filtration angle of the eye is avoided.

Iris fixated IOLs are disclosed in United States patents 4,215,440 and 5,192,319 to Jan Worst. Both of such patents disclose IOLs employing one or more optic fixation members formed having a pair of pincer arms which, acting together, pinch an anterior surface region of the iris. This pinching action detachably attaches the IOL to the iris so that the IOL optic is ideally fixated in the region of the iris opening (i.e., the pupil of the eye).

However, the present inventor considers that improvements to the iris fixated IOL designs disclosed in the two above-cited Worst patents are desirable, particularly in the areas of improving optic centration and enabling small incision implanting.

US4,542,541 (Pannu) discloses a universal intraocular lens having snag-resistent looped supports.

GB2085731 (Tennant) discloses an implant lens with biarcuate fixation wherein the implanted weight is distributed over a large area to minimise the localisation of support forces.

### SUMMARY OF THE INVENTION

In accordance with a first aspect of the present invention, there is provided an iris fixated intraocular lens which comprises an optic having an optical axis and anterior and posterior sides and at least two fixation members, and which may have an overall diameter of between about 7.5 mm and about 10 mm. Each of the fixation members has a proximal end region and a distal end region, the proximal end region comprising a flexible strand, preferably, a single flexible strand, fixed to an edge region of the optic so as to extend generally tangentially outwardly therefrom. The distal end region is formed into a loop having defined therein at least one narrow iris pincher gap which has a substantially uniform width and is sized for pinching a small surface segment of iris tissue into said gap for detachably attaching said intraocular lens to an iris anterior surface.

In a preferred embodiment of the invention, the at least one pincher gap is located on a line generally perpendicular to the optical axis, but may alternatively be formed at an angle to the perpendicular line.

It is preferred that the at least two fixation members include first and second fixation members that are substantially identical to one another and are attached to the optic on opposite sides of the optical axis. The first and second fixation members are constructed separately from the optic, the intraocular lens being thereby a three-piece intraocular lens.

It is further preferred that the optic is constructed from an elastically deformable material, which may be a silicone material or an acrylic material. Also, the at least two fixation members lie in an at least substantially common plane located posterior of the optic.

The distal end loop of each of the at least two fixation members may be elongated into a curved shape, and in some embodiments of the invention, each of the distal end loop includes means dividing the loops into first and second segments; in which case, a first pincher gap is defined in the first loop segment and a second pincher gap is defined in the second loop segment. Preferably, the loop dividing means lies generally perpendicular to the optical axis of the optic.

The at least one pincher gap preferably has a width of between about 0.05 mm and about 0.25 mm, and preferably has a length between about 0.2 mm and about 0.5 mm. The pincher gap in the distal end loop of each of the first and second fixation members may be located in a region of the loop closest to said optical axis, or in a region of the loop furthest from said optical axis. In either case, the pincher gaps are spaced a preferred distance, D, between about 8.0 mm and about 9.0 mm, away from the optical axis of the optic.

Because the fixation members are constructed as a flexible strand and the optic is constructed from an elastically deformable material, the resulting three-piece iris fixated IOL of the invention can be folded, rolled or otherwise deformed for insertion through a small, sutureless incision in the eye, as is highly desirable for such reasons as minimal patient trauma and the reduced possibility of surgical complications. Also importantly, the flexible strand fixation members enable accurate centration of the associated optic in the patient's eye upon fixation of the IOL to the iris.

### BRIEF DESCRIPTION OF THE DRAWINGS

The present invention can be more readily understood by a consideration of the following detailed description when taken in conjunction with the accompanying drawings, in which:
FIG. 1 is a vertical cross sectional drawing of forward regions of a representation human eye, showing the cornea, iris and natural lens and showing an iris fixated intraocular lens (IOL) of the present invention implanted in the anterior chamber of the eye and fixed to the anterior surface of the iris;
FIG. 2 is a front view of one embodiment of a three piece iris fixated IOL of the present invention, showing the optic and an opposing pair of optic support or fixation members (haptics), each terminating in an elongated fixation loop having a narrow pincher gap for enabling detachable attachment of the IOL to the anterior surface of a patient's iris, the pincer gaps being shown directly facing the optic;
FIG. 3 is a side view of the IOL of FIG. 2, showing forward vaulting of the optic relative to the fixation loops;
FIG. 4 is a partial front view of a variation fixation loop corresponding to the fixation loops shown in FIG. 2. showing a spaced apart pair of iris pincher gaps defined in the elongated vertically-clivided fixation loop, both of such gaps shown directly facing the optic;
FIG, 5 is a front view of a variation three piece iris fixated IOL of the present invention, showing the optic and an opposing pair of haptics, each such haptic shown curving closely around the optic and terminating in an elongated fixation loop having a narrow, perpendicular pincher gap for enabling detachable attachment of the IOL to a patient's iris, the pincher gaps being shown facing away from the optic;

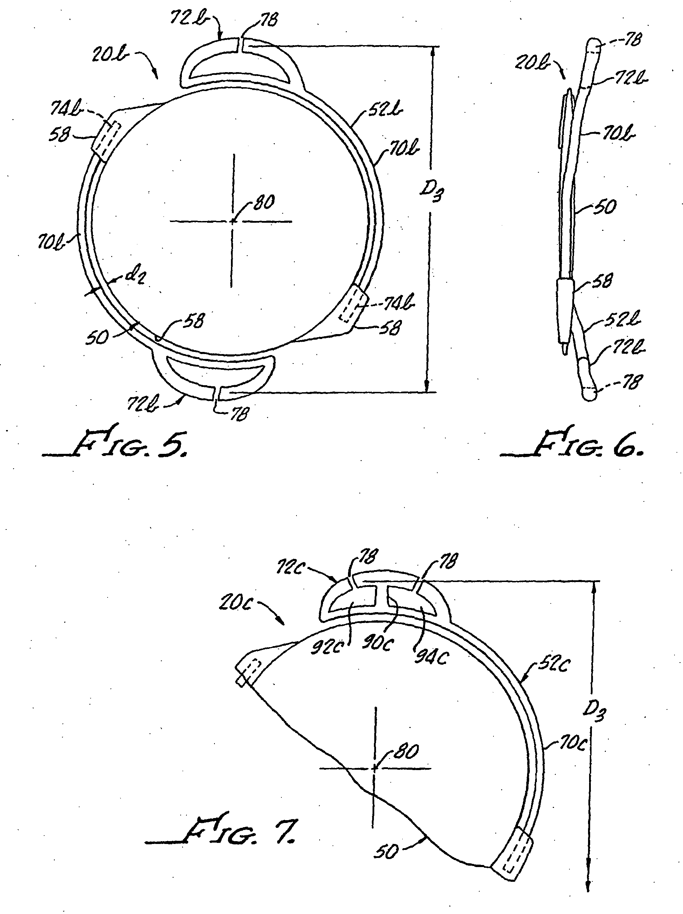

FIG. 6 is a side view of the IOL of FIG. 5, showing forward vaulting of the optic relative to the fixation loops;

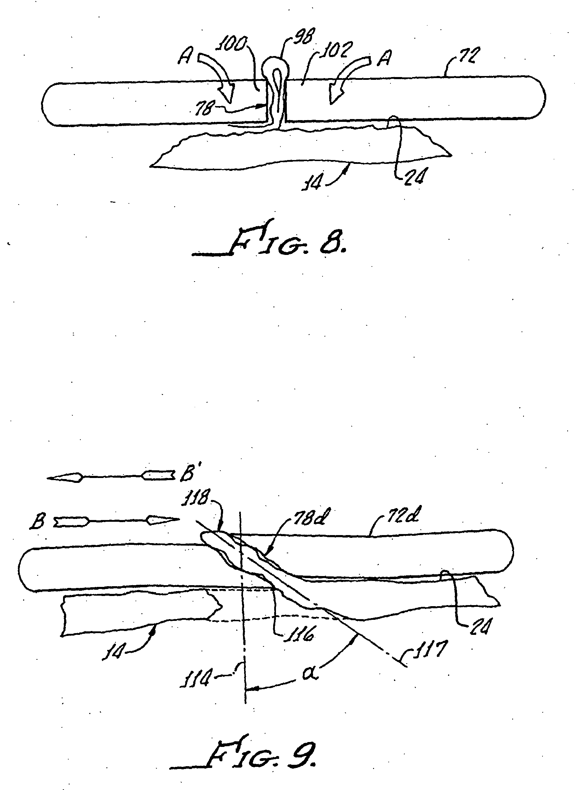

FIG. 7 is a partial front view of a variation fixation loop corresponding to the fixation loops shown in FIG. 5, showing a spaced apart pair of iris pincher gaps defined in the elongated vertically-divided fixation loop, both of such gaps shown directed away from the optic;
FIG. 8 is a drawing depicting the manner in which a representative right angle pincher gap, such as those shown in FIGS. 2, 4, 5 and 7, is operative for pinching an anterior surface region of an iris in a manner detachably attaching the associated fixation loop and thus the associated IOL to the iris; and
FIG. 9 is a drawing depicting the manner in which a representative angled pincher gap, corresponding to the right angle pincer gaps shown in FIGS. 2, 4, 5 and 7 is used to engage the anterior surface of an iris in a manner detachably attaching the associated fixation loop and thus the associated IOL to the iris.

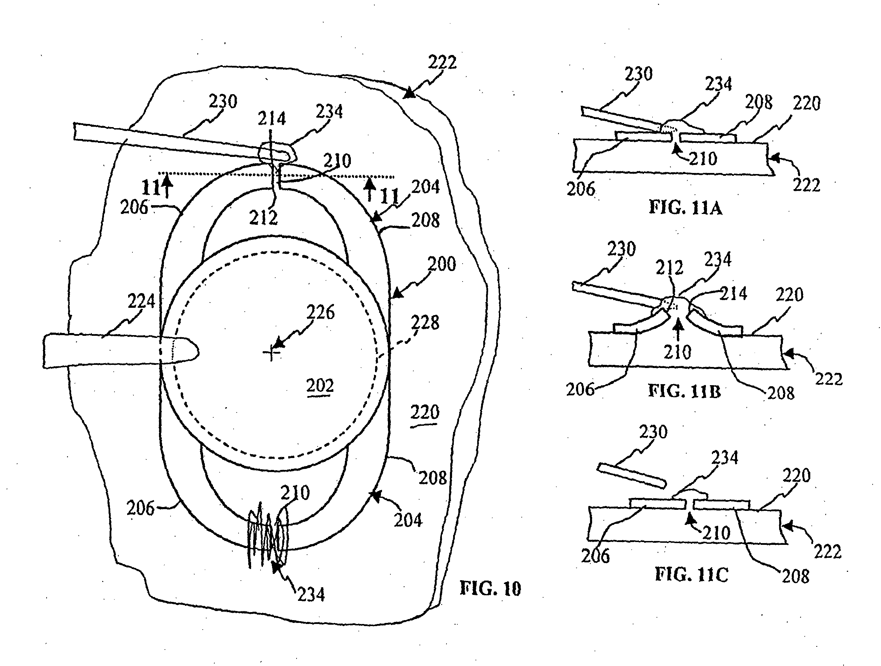

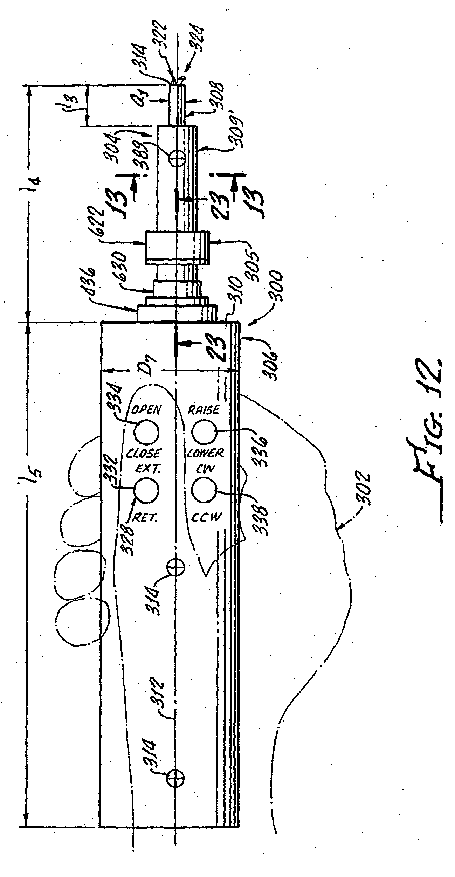

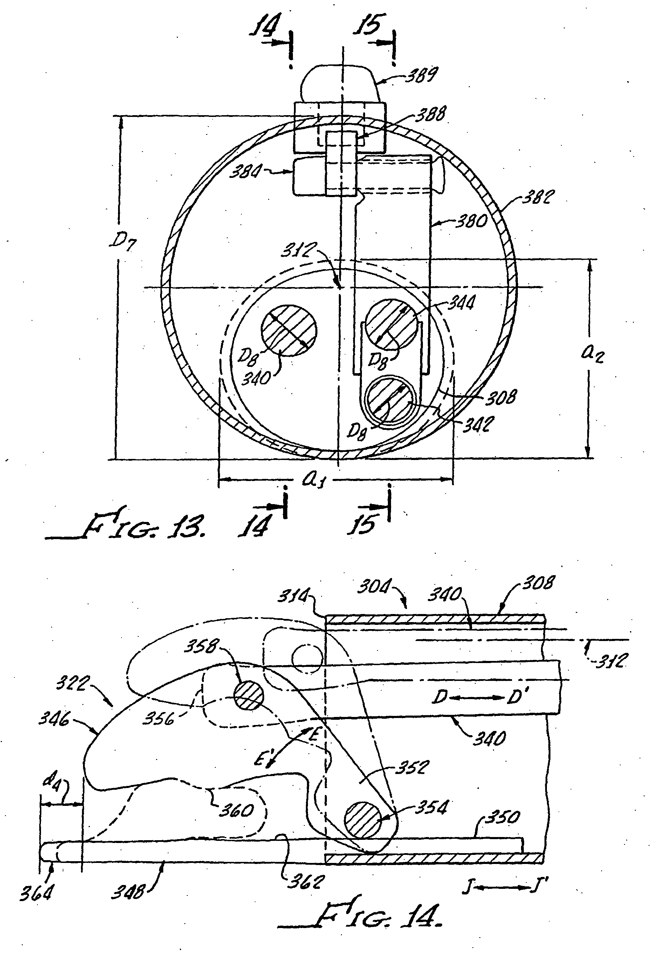

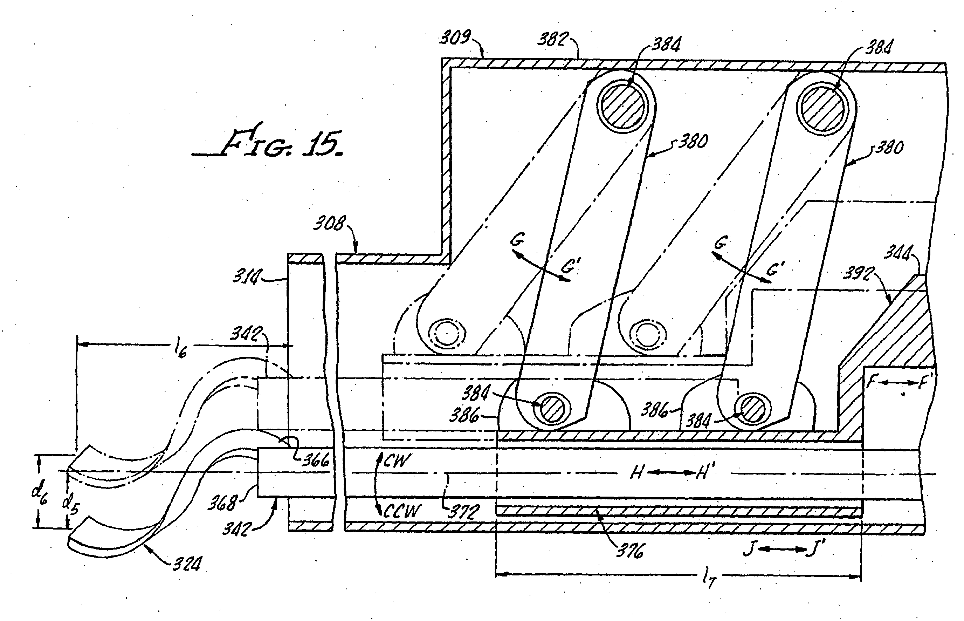

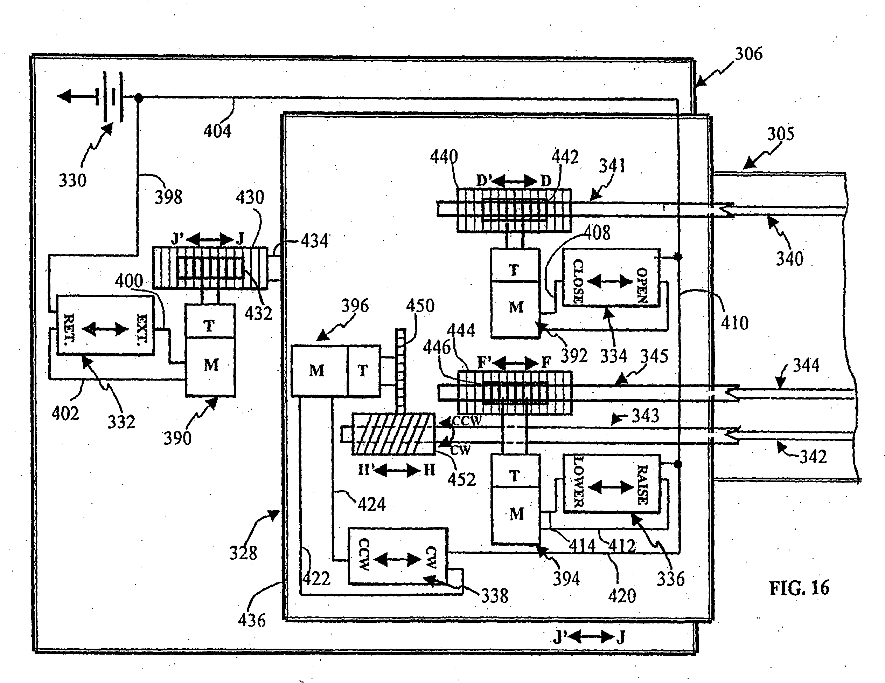

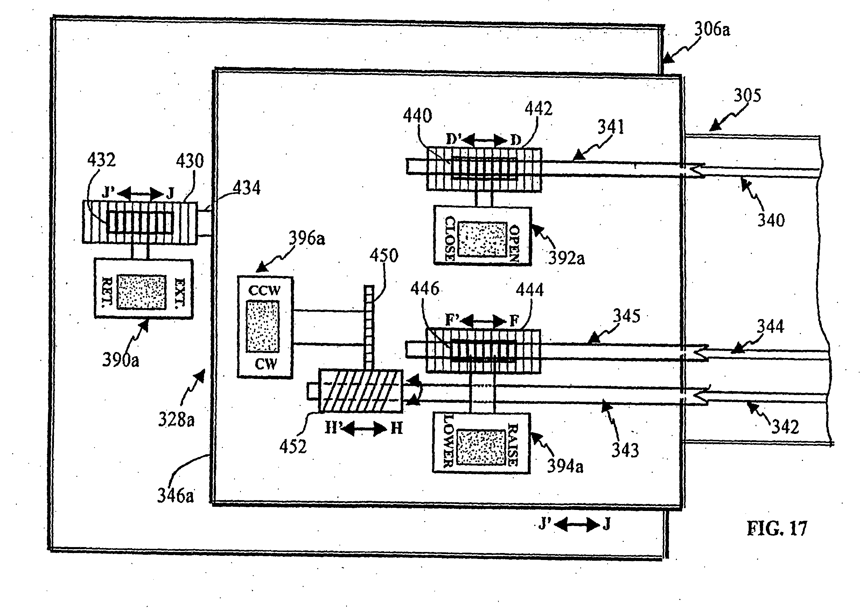

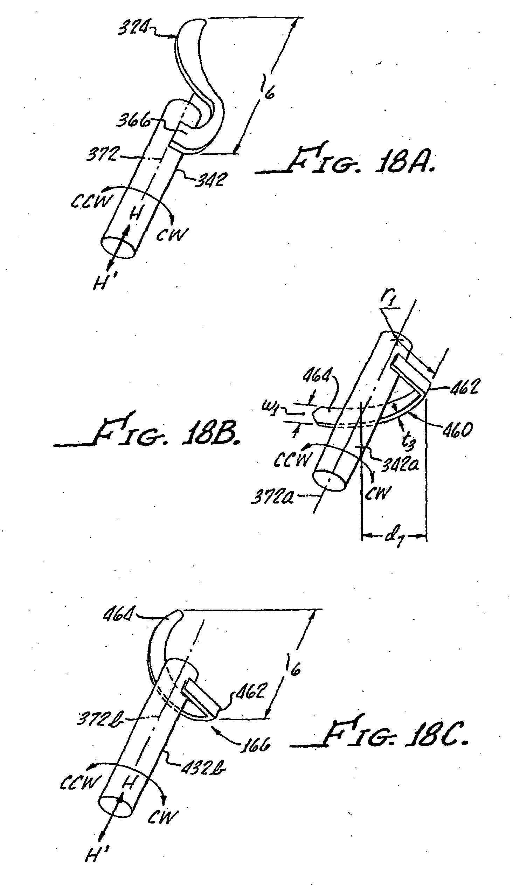

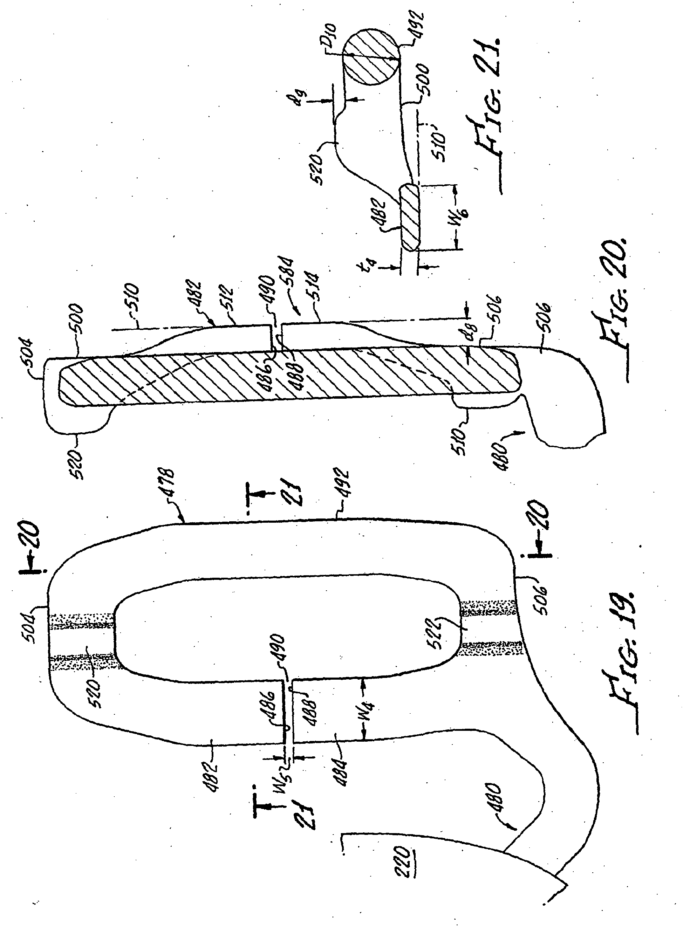

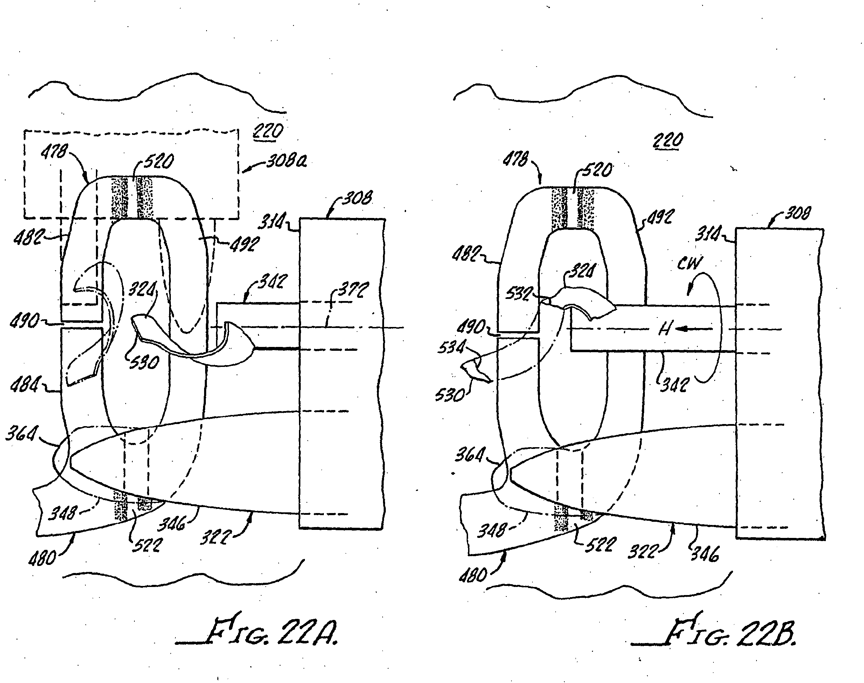

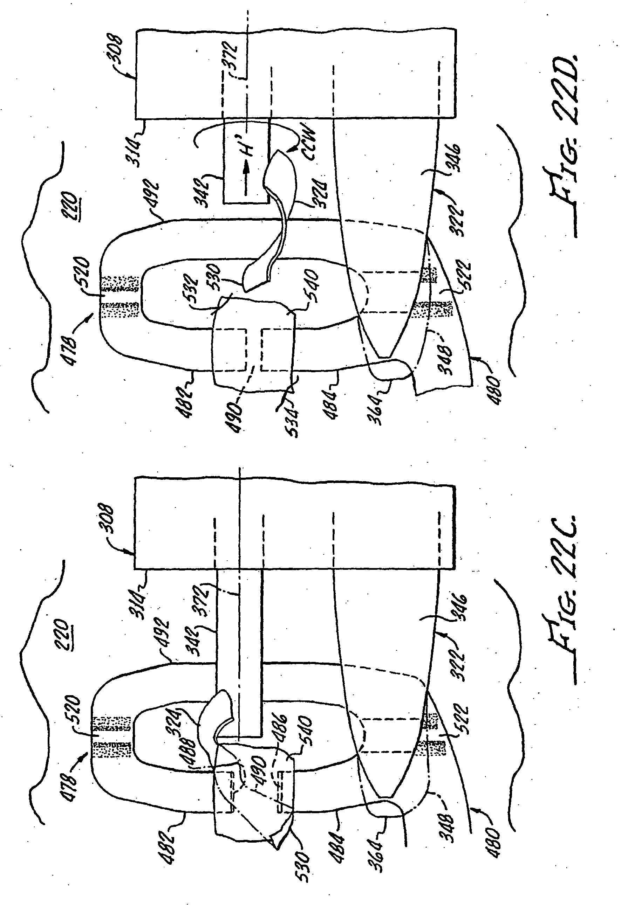

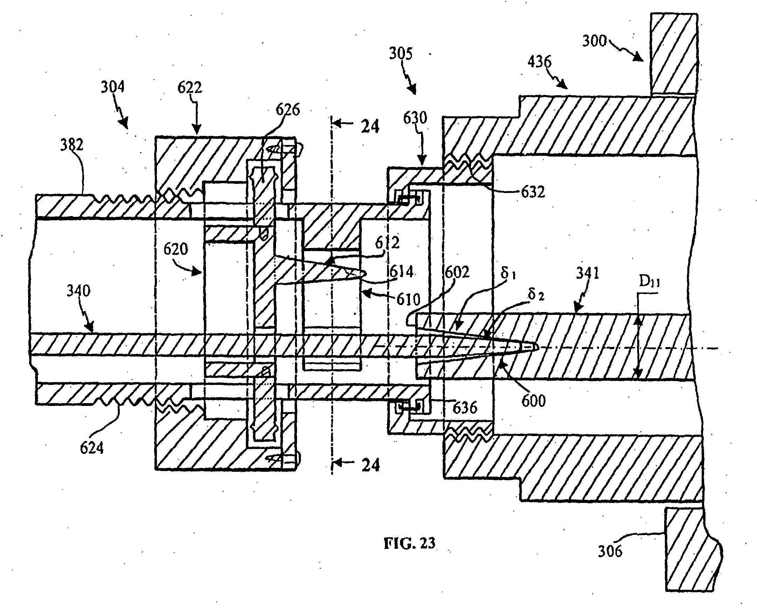

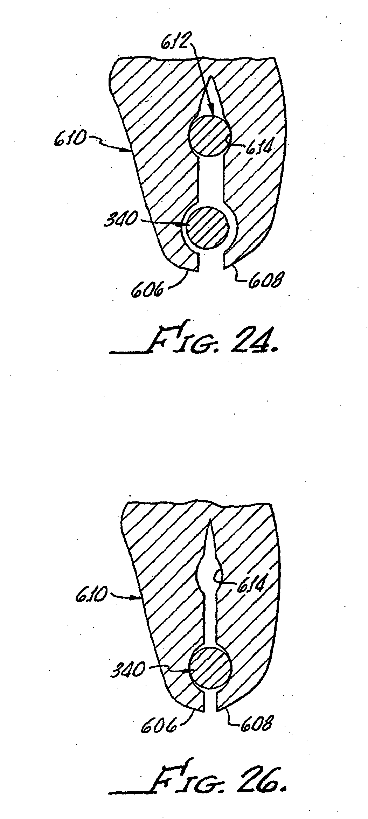

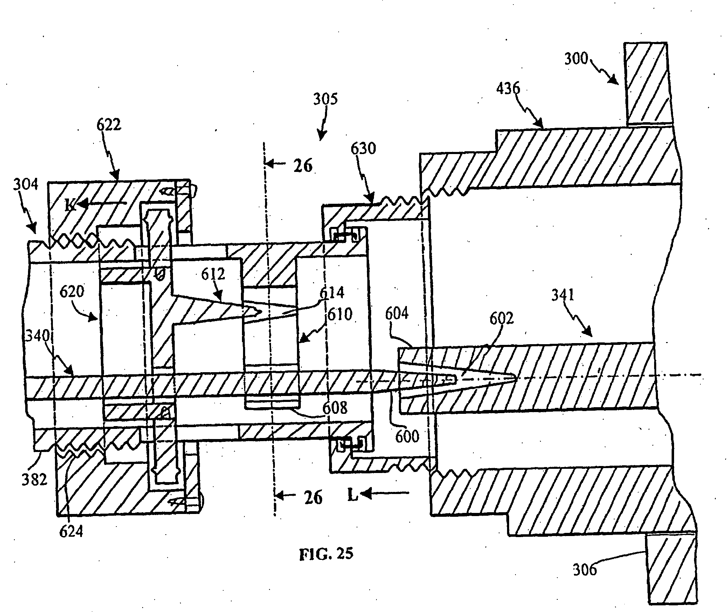

FIGS. 10 to 25 illustrate various preferred embodiments of a combination forceps and enclavation needle, and its method of use, by way of example only.

In the various FIGS., the same elements and features are given the same reference numbers. In the various embodiments, corresponding elements and features are given the same reference numbers as first set forth, followed by an "a". "b", "c", and so on, as appropriate and as will be evident in the following DESCRIPTION.

### Description of the Preferred Embodiment

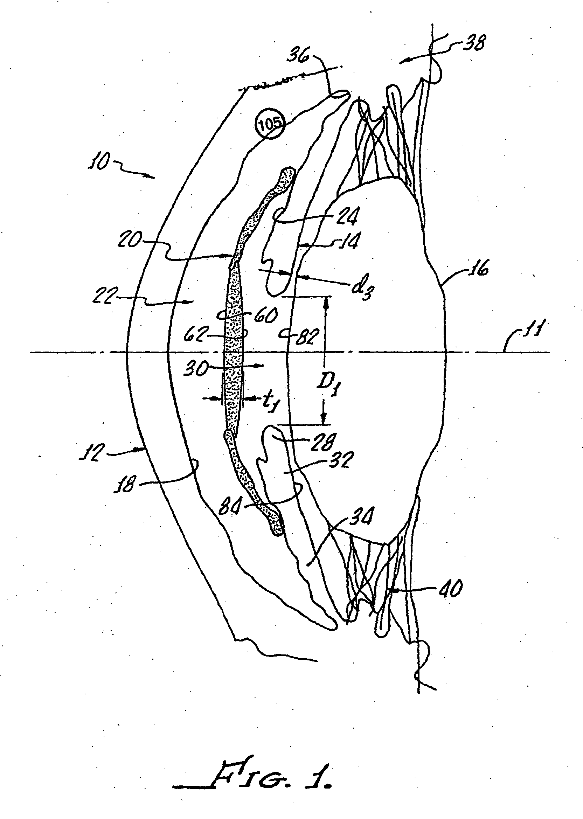

There is shown in FIG. 1, in vertical cross section, a forward region 10 of a representation human eye having an optical axis 11 (Axis of symmetry). Depicted in the FIG. are a cornea 12, an iris 14 and an intact, natural crystalline lens 16. A (posterior) comeal endothelium surface 18 is identified on cornea 12.

An iris fixated intraocular lens (IOL) 20, according to a preferred embodiment of the present invention, is shown implanted in an anterior chamber 22 of eye region 10 (posterior to corneal endothelium surface 18) and fixated, in a manner described below, to an anterior surface 24 of iris 14.

Identified in FIG. 1, to facilitate the understanding of the present invention, is an annular pupiliary spincter region 28 of iris 14 that surrounds and controls a pupil or pupiliary opening 30 having a diameter, D₁, that typically no greater than about 8 mm for normal vision.

Further identified are an annular iris collarette region 32 and an annular pupiliary dilator muscle region 34 of iris 14. An annular chamber angle 36 is identified at a peripheral edge region of iris 14, as is an annular trabecular meshwork 38. An annular ciliary processes 40 is indicated at the peripheral attachment of natural lens 16.

As is further depicted in FIG. 1, iris fixated IOL 20 is fixated to iris anterior surface 24 in the general region of iris collarette 32 (the thickest region of iris 14), radially outwardly from pupillary sphincter 28.

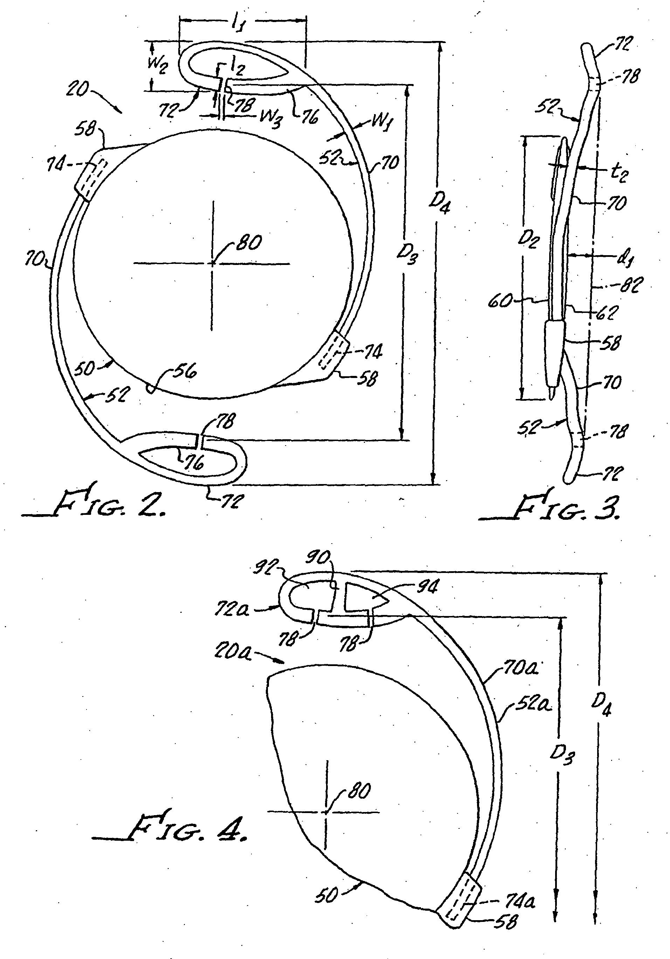

Shown in FIGS 2 and 3, comprising iris fixated IOL 20 are an optic 50 and a pair of opposing, similar and preferably identical, fixation members or haptics 52. Projecting sidewardly (radially) from opposite sides of a peripheral edge 56 of optic 50, and preferably formed in one piece with the optic, are similar structural haptic attachment abutments or bosses 58. Optic 50, which has respective anterior and posterior surfaces 60 and 62 (FIGS. 1 and 3), may be constructed as convex-convex (as depicted in FIG. 1), convex-concave, convex-planar, or concave-planar or concave-concave, all such and other configurations being within the scope of the present invention. Optic 50 may advantageously be provided in the diopter range between about -15 and about +15.

It is preferred that optic 50 be constructed from an elastically deformable material, such as a silicone or acrylic material, enabling the optic to be folded, rolled or otherwise deformed so that IOL 20 can be implanted through an ocular incision no larger than about 3.5 mm. It is therefore preferable that the material from which optic 50 is contructed has an index of refraction of at least about 1.46 and that the optic has a diameter, D₂, of between about 5.5 mm and about 7.0 mm (FIG. 3) and a center thickness, t₁, no greater than about 0.8 mm (FIG. 1).

Each of haptics 52, which are preferably constructed (as by micro-machining) from polymethyl methacrylate (PMMA), is formed having an arcuate, flexible proximal end region 70 and a generally flat, loop-shaped distal end region 72. A proximal end 74 of each haptic 52 is fixed into an associated one of bosses 58 (FIG. 2) so that haptic proximal end region 70 extends in a direction tangential to optic edge 56. Such haptic-to-optic fixation can be of any type used by IOL manufacturers for the secure attachment of haptics to soft, flexible optics.

Haptic proximal end region 70 is arcuate in plan view and arches away from optic 50 (FIG. 2). Further, proximal end region 70 is made flexible, particularly in a plane parallel to the plane of optic 50, by preferably having a width, w₁, of about 0.25 mm and a thickness, t₂ (FIG. 3) of about 0.35 mm. Preferably portions of haptic 52 defining distal end region loop 72 have about the same thickness as set forth for haptic proximal end region 70, and may be somewhat wider, as set forth below.

The loop into which haptic distal end region 72 is formed may be of a variety of shapes. However, the end region loop is shown in FIG. 2 as being elongated into a curved shaped having a length, I₁ and flattened into a width, w₂. By way of example, with no limitation intended or implied, such loop length, I₁, may be about 3.0 mm and such loop width, w₂, may be about 1.0 mm.

A side region 76 of distal end region loop 72 that is closest to and directly faces optic 50 is formed defining an iris pincher gap 78 (FIG. 2) having a width, w₃, of about 0.1 mm and a length, I₂, of about 0.4 mm. Iris pincher gap 78 is shown oriented in a radial direction relative to a center 80 of optic 50, but may alternatively be oriented in another direction. As further, shown in FIG 2, both iris pincher gaps 78 of the two haptics 52 are centered on a diameter, D₃, which is preferably about 8.5 mm. Pincher gaps 78 of both haptics 52 also lie generally on a common plane 82 (FIG 3), the haptics being arched so that optic 50 is vaulted forwardly into anterior chamber 22 (FIG. 1) with posterior surface 62 anterior of plane 82 by a distance, d₁, that is preferably about 0.5 mm.

Overall diameter, D₄ of IOL 20 (to ends of haptics 52) is preferably between about 7-5 mm and about 10 mm so that the IOL haptics engage iris 14 at iris collarette region 32, as noted above (FIG. 1).

As a result of the flexibility of haptics 52, after one haptic has been attached to iris 14 by a pinching action (more particularly described below), optic center 80 can be easily aligned with optical axis 11 by flexing of the second haptic 52 before the second haptic is attached to the iris. Thus, centration of optic 50 on optical axis 11 of the eye is easily achieved.

Moreover, with optic 60 constructed from an elastically deformable material, IOL 20 can be implanted through a small ocular incision, as is important to minimise surgical trauma and possible complications, and reduce patient recovery time, all as compared to the surgical procedure required to implant a rigid iris fixation IOL. Further in this regard, the explanting of the flexible IOL 20, in the event explanting becomes necessary as the patient's vision changes with time, is also made easier.

From the foregoing, it will be appreciated that many variations to IOL 20 and particularly to haptics 52 which attach the IOL to iris 14 are possible and are to be considered as being covered by the present invention.

### IOL VARIATION OF FIG. 4:

One of such variations is shown in FIG. 4 in connection with a variation IOL 20a, which is identical for descriptive purposes to above-described IOL 20 except as otherwise particularly described below. Corresponding elements and features are given the same reference numbers set forth above followed by an "a".

As shown, a looped distal end region 72a of a haptic 52a (corresponding to haptic 52) is divided radially (relative to center 80 of optic 50) by a narrow wall 90 into respective first and second loop sectors 92 and 94. Each such sector 92 and 94 is constructed to define an iris pincher gap 78 directly facing optic 50. Thus, each haptic 52a (only a representative one of which is shown) incorporates in distal end region 72a a spaced-apart pair of iris pincher gaps 78. This described doubling of the number of iris pincher gaps 78 in haptic loops 72a may sometimes be advantageous in securely detachably fixing IOL 20a to iris 14.

### IOL VARATION OF FIGS- 5 AND 6:

Another such variation is shown in FIGS. 5 and 6 in connection with a variation iris fixation IOL 20b, which is identical for descriptive purposes to above-described IOL 20 except as otherwise particularly described below. Corresponding elements and features are given the same reference numbers set forth above followed by a "b".

A principal distinction between IOL 20b and above-described IOL 20 relates to pincher gaps 78 on haptic loops 72b facing away from optic 50 instead of directly facing the optic in the case of the above-described IOL 20. Because pincher gaps 78 are spaced apart the same distance, D₃ (before disclosed in connection with IOL 20), proximal regions 70b of haptics 52b curve more closely around optic 50. Haptics 52b,are generally spaced from optic edge 56 a distance, d₂, that is at least about equal to a closest separation distance, d₃ (FIG. 1), between anterior surface 82 of natural lens 16 and posterior surface 84 of iris 14 (a distance typically of about 0.3 mm).

Since haptics 52b are otherwise similar to above-described haptics 52, this increased, C-curvature of haptics 52b may provide somewhat increased haptic flexibility. Moreover, orienting pincher gaps 78 on haptic loops 72b away from optic 50 may, in some instances, facilitate fixation of the IOL to iris 14. The vaulting of optic 50 relative to haptic loops 72b is preferably the same as disclosed above relative to IOL 20.

### IOL VARIATION OF FIG. 7:

FIG. 7 depicts another variation iris fixated IOL 20c, which is identical for descriptive purposes to above-described IOL 20b except as otherwise particularly described below. Previously described features and elements are given the same reference number followed by a "c".

As can be seen, IOL 20c combines the described double pincher gap features shown for IOL 20a in FIG. 4 with IOL 20b (FIGS 5 and 6.). Thus, as shown in FIG. 7, representative haptic loop 72c is vertically divided by a narrow wall 90c into first and second loop sectors 92c and 94c, respectively. Each sector 92c and 94c has defined a pincher gap 78 that faces away from associated optic 50.

Pincher gaps 78 on both haptic loops 72c (only one such loop being shown) are spaced a distance D₃ (defined above) apart.

### OPERATION OF PINCHER GAPS:

FIG. 8 depicts the manner in which a representative one of pincher gaps 78, on a representative haptic distal end region loop 72 pinches up a small surface segment 98 of iris tissue into the gap, thereby releasably or detachably fixing the associated haptic (e.g., haptic 52), and hence the associated IOL (e.g., IOL 20), to iris 14.

This pinching up of iris segment 98 is accomplished, for example, by deflecting haptic loop regions 100 and 102 on each side of gap 78, downwardly (direction of Arrows "A") into iris surface 24. When the loop regions are released, they return to their original shape, thereby trapping iris segment 98 in gap 78.

### VARIATION IRIS PINCER GAP OF FIG. 9:

It is to be understood that variations of the iris pincher gap may be made within the scope of the present invention and used in place of above-described pincher gap(s) 78. An example of such a variation is depicted in FIG. 9, in which a slanted iris pincher gap 78d (corresponding to above-described pincer gap 78) is depicted formed or defined in a representative haptic distal end region loop 72d (corresponding to above-described distal end region loop 72).

Pincher gap 78d is depicted in FIG. 9 as formed or defined along a line 112 that is at an angle, α, relative to a line 114 perpendicular to end region loop 72d. Preferably, slant angle, α, is between about 30 degrees and about 60 degrees, with a slant angle of about 45 degrees being most preferred.

It is evident from FIG. 9 that when end region loop 72d is pressed against iris anterior surface 24 and is pushed or advanced in the direction indicated by Arrow "B", a sharp, leading lower edge 116 at gap 78d cuts into iris 14. This causes a small sliver 118 of iris 14 to be extruded into gap 78d, to thereby detachably fixate end region loop 72d, and hence associated haptic and IOL (neither shown in FIG. 9) to iris 14.

Distal end region loop 72d can be detached from iris by merely rotating the end region loop back In the direction indicated by Arrow "B".

The various features of the illustrated embodiments of the combination forceps and enclavation needle and its method of use, will be apparent to those skilled in the art from the foregoing description and from Figures 10 to 25.

Although there have been described above an iris fixated IOL, and variations thereof, in accordance with the present invention for purposes of illustrating the manner in which the present invention may be used to advantage, it is to be understood that the invention is not limited thereto and includes any and all variations and equivalent arrangements which fall within the scope of the appended claims.

## Claims

1. An iris fixed intraocular lens (20) which comprises:
a. an optic (50) having an optical axis and anterior and posterior sides (60,62); and
b. at least two fixation members (52), each of said fixation members having a proximal end region (70) and a distal end region (72), said proximal end region comprising a flexible strand fixed to an edge region of said optic so as to extend generally tangentially outwardly therefrom, and said distal end region being formed into a loop **characterised in that** said loop has defined therein at least one narrow iris pincher gap (78) which has a substantially uniform width and is sized for pinching a small surface segment of iris tissue into said gap for detachably attaching said intraocular lens to an iris anterior surface.

2. The iris fixated intraocular lens as claimed in claim 1, wherein said at least one pincher gap is located on a line generally perpendicular to said optical axis and is positioned at a radius from said optical axis of about 4.25 mm.

3. The iris fixated intraocular lens as claimed in claim 2, wherein said first and second fixation members are constructed separately from said optic, said intraocular lens being thereby a three-piece intraocular lens.

4. The iris fixated intraocular lens as claimed in Claim 3, wherein said optic is constructed from an elastically deformable material.

5. The iris fixated intraocular lens as claimed in Claim 4, wherein said elastically deformable material is a silicone material or an acrylic material.

6. The iris fixated intraocular lens as claimed in Claim 1, wherein the distal end loops of each of said at least two fixation members lie in an at least substantially common plane located posteriorally of said optic.

7. The iris fixated intraocular lens as claimed in Claim 1, wherein said distal end loop of each of said at least two fixation members is elongated into a curved shape.

8. The iris fixated intraocular lens as claimed in claim 1, wherein:
the distal end loop of each of said at least two fixation members is elongated into a curved shape, each said distal end loop including means dividing said loops into first and second segments.

9. The iris fixated intraocular lens as claimed in claim 8, wherein said dividing means lies generally perpendicular to said optical axis of said optic.

10. The iris fixated intraocular lens as claimed in Claim 8, including a first pincher gap defined in said first loop segment and a second pincher gap defined in said second loop segment.

11. The iris fixated intraocular lens as claimed in Claim 1, wherein said pincher gap is defined at an angle to said perpendicular line.

12. The iris fixated intraocular lens as claimed in Claim 1, wherein said at least one pincher gap has a width of between about 0.05 mm and about 0.25 mm, or wherein said at least one pincher gap has a length of between about 0.2 mm and about 0.5 mm.

13. The iris fixated intraocular lens as claimed in Claim 1, wherein the intraocular lens has an overall diameter of between about 7.5 mm and about 10 mm.

14. The iris fixated intraocular lens as claimed in claim 1, wherein said flexible strand is a single flexible strand.

15. The iris fixated intraocular lens as claimed in Claim 14, wherein said first and second fixation members are substantially identical to one another and are attached to said optic on opposite sides of said optical axis.

16. The iris fixated intraocular lens as claimed in Claim 14, wherein the distal end loops of each of said first and second fixation members lie in a generally common plane, and wherein said pincher gaps are located on a diameter, D, through the optical axis of said optic.

17. The iris fixated intraocular lens as claimed in Claim 16, wherein said distance, D, is between about 8.0 mm and about 9.0 mm.

18. The iris fixated intraocular lens as claimed in Claim 14, wherein said distal end loop of each of said first and second fixation members is elongated into a curved shape having a major axis generally parallel to said optical axis.

19. The iris fixated intraocular lens as claimed in Claim 14, wherein the pincher gap in the distal end loop of each of said first and second fixation members is located in a region of said loop closest to said optical axis.

20. The iris fixated intraocular lens as claimed in claim 1 wherein said fixation members comprise first and second fixation members; wherein said flexible strand is a single flexible strand and wherein said narrow iris pincher gap is located on a line generally perpendicular to said optical axis, the pincher gap in the distal end loop of each of said first and second fixation members being located in a region of said loop furthest from said optical axis.

21. The iris fixated intraocular lens as caimed in claim 1 wherein: said fixation members comprise first and second fixation members; said flexible strand is a single flexible strand; and wherein said narrow iris pincher gap is located on a line generally perpendicular to said optical axis, each of said distal end loops including means dividing the loop into first and second loop segments, said dividing means lying along a line generally perpendicular to said optical axis, and including a first pincher gap defined in said first loop segment and a second pincher gap defined in said second loop segment.

22. The iris fixated intraocular lens as claimed in Claim 14, wherein said pincher gap has a width of between about 0.05 mm and about 0.25 mm and a length of between about 0.2 mm and about 0.5 mm.

23. The iris fixated intraocular lens as claimed in claim 1 wherein the lens is a three-piece lens;
wherein said optic is constructed from an elastically deformable plastic material;
and wherein said fixation members comprise first and second fixation members; and
wherein said flexible strand comprises a single flexible strand.

24. The three-piece iris fixated intraocular lens as claimed in Claim 23, wherein said first and second fixation members are substantially identical to one another and are attached to said optic on opposite sides of said optical axis.

25. The three-piece iris fixated intraocular lens as claimed in Claim 23, wherein said at least one pincher gap in the distal end loop of each of said first and second fixation members is located in a region of said loop closest to said optical axis.

26. The iris fixated intraocular lens as claimed in claim 23,
wherein said at least one pincher gap in the distal end loop of each of said first and second fixation members is located in a region of said loop furthest from said optical axis.

## Patentansprüche

1. An einer Iris zu fixierende intraokulare Linse (20), die aufweist:
a. Eine Optik (50) mit einer optischen Achse und einer vorderen und einer hinteren Seite (60, 62) und
b. mindestens zwei Befestigungselemente (52), von denen jedes einen proximalen Endbereich (70) und einen distalen Endbereich (72) hat, wobei der proximale Endbereich einen flexiblen Strang aufweist, der so an einem Randbereich der Optik befestigt ist, dass er sich im Wesentlichen tangential von diesem nach außen erstreckt, und der distale Endbereich in eine Schlaufe ausgebildet ist, **dadurch gekennzeichnet, dass** in der Schlaufe mindestens ein schmaler Iriseinklemmspalt (78) ausgebildet ist, der eine im Wesentlichen gleichförmige Breite hat und dimensioniert ist, um ein kleines Oberflächensegment von Irisgewebe zur lösbaren Befestigung der intraokularen Linse an einer vorderen Oberfläche einer Iris in dem Spalt einzuklemmen.

2. An einer Iris zu fixierende intraokulare Linse nach Anspruch 1, bei der der mindestens eine Einklemmspalt auf einer Linie angeordnet ist, die im Wesentlichen senkrecht zu der optischen Achse ist, und bei einem Radius von der optischen Achse von ungefähr 4,25 mm angeordnet ist.

3. An einer Iris zu fixierende intraokulare Linse nach Anspruch 2, bei der das erste und das zweite Befestigungselement separat von der Optik ausgebildet sind, und die intraokulare Linse auf diese Weise eine dreiteilige intraokulare Linse ist.

4. An einer Iris zu fixierende intraokulare Linse nach Anspruch 3, bei der die Optik aus einem elastisch verformbaren Material ausgebildet ist.

5. An einer Iris zu fixierende intraokulare Linse nach Anspruch 4, bei der das elastisch verformbare Material ein Silikonmaterial oder ein Acrylmaterial ist.

6. An einer Iris zu fixierende intraokulare Linse nach Anspruch 1, bei der die am distalen Ende angeordneten Schlaufen von jedem der mindestens zwei Befestigungselemente in einer zumindest im Wesentlichen gemeinsamen Ebene liegen, die hinter der Optik angeordnet ist.

7. An einer Iris zu fixierende intraokulare Linse nach Anspruch 1, bei der die am distalen Ende angeordnete Schlaufe von jedem der mindestens zwei Befestigungselemente in eine gekrümmte Form gestreckt ist.

8. An einer Iris zu fixierende intraokulare Linse nach Anspruch 1, bei der:
Die am distalen Ende angeordnete Schlaufe von jedem der mindestens zwei Befestigungselemente in eine gekrümmte Form gestreckt ist, wobei jede der an einem distalen Ende angeordneten Schlaufen ein Mittel enthält, das die Schlaufen in ein erstes und ein zweites Segment unterteilt.

9. An einer Iris zu fixierende intraokulare Linse nach Anspruch 8, bei der das Unterteilungsmittel im Wesentlichen senkrecht zu der optischen Achse der Optik liegt.

10. An einer Iris zu fixierende intraokulare Linse nach Anspruch 8, die einen ersten Einklemmspalt, der in dem ersten Schlaufensegment ausgebildet ist, und einen zweiten Einklemmspalt enthält, der in dem zweiten Schlaufensegment ausgebildet ist.

11. An einer Iris zu fixierende intraokulare Linse nach Anspruch 1, bei der der Einklemmspalt in einem Winkel zu der senkrechten Linie ausgebildet ist.

12. An einer Iris zu fixierende intraokulare Linse nach Anspruch 1, bei der der mindestens eine Einklemmspalt eine Breite von zwischen ungefähr 0,05 mm und ungefähr 0,25 mm hat oder bei der der mindestens eine Einklemmspalt eine Länge von zwischen ungefähr 0,2 mm und ungefähr 0,5 mm hat.

13. An einer Iris zu fixierende intraokulare Linse nach Anspruch 1, bei der die intraokulare Linse einen Gesamtdurchmesser von zwischen ungefähr 7,5 mm und ungefähr 10 mm hat.

14. An einer Iris zu fixierende intraokulare Linse nach Anspruch 1, bei der der flexible Strang ein einzelner flexibler Strang ist.

15. An einer Iris zu fixierende intraokulare Linse nach Anspruch 14, bei der das erste und das zweite Befestigungselement im Wesentlichen identisch zueinander sind und an entgegengesetzten Seiten der optischen Achse an der Optik befestigt sind.

16. An einer Iris zu fixierende intraokulare Linse nach Anspruch 14, bei der die am distalen Ende angeordneten Schlaufen von jedem des ersten und des zweiten Befestigungselements in einer im Wesentlichen gemeinsamen Ebene liegen und bei der die Einklemmspalte auf einem Durchmesser D durch die optische Achse der Optik angeordnet sind.

17. An einer Iris zu fixierende intraokulare Linse nach Anspruch 16, bei der der Abstand D zwischen ungefähr 8,0 mm und ungefähr 9,0 mm beträgt.

18. An einer Iris zu fixierende intraokulare Linse nach Anspruch 14, bei der die am distalen Ende angeordnete Schlaufe von jedem des ersten und des zweiten Befestigungselements in eine gekrümmte Form gestreckt ist, die eine Hauptachse im Wesentlichen parallel zu der optischen Achse hat.

19. An einer Iris zu fixierende intraokulare Linse nach Anspruch 14, bei der der Einklemmspalt in der am distalen Ende angeordneten Schlaufe von jedem des ersten und des zweiten Befestigungselements in einem Bereich der Schlaufe angeordnet ist, der sich am dichtesten an der optischen Achse befindet.

20. An einer Iris zu fixierende intraokulare Linse nach Anspruch 1, bei der die Befestigungselemente ein erstes und ein zweites Befestigungselement umfassen,
wobei der flexible Strang ein einzelner flexibler Strang ist und wobei sich der schmale Iriseinklemmspalt auf einer Linie befindet, die im Wesentlichen senkrecht zu der optischen Achse ist, wobei der Einklemmspalt in der am distalen Ende angeordneten Schlaufe von jedem des ersten und des zweiten Befestigungselements in einem Bereich der Schlaufe angeordnet ist, der am weitesten von der optischen Achse entfernt ist.

21. An einer Iris zu fixierende intraokulare Linse nach Anspruch 1, bei der: Die Befestigungselemente ein erstes und ein zweites Befestigungselement umfassen, der flexible Strang ein einzelner flexibler Strang ist und bei der sich der schmale Iriseinklemmspalt auf einer Linie befindet, die im Wesentlichen senkrecht zu der optischen Achse ist,
wobei jede der an einem distalen Ende angeordneten Schlaufen ein Mittel enthält, das die Schlaufe in ein erstes und ein zweites Schlaufensegment unterteilt, wobei das Unterteilungsmittel entlang einer Linie liegt, die im Wesentlichen senkrecht zu der optischen Achse ist, und einen ersten Einklemmspalt, der in dem ersten Schlaufensegment ausgebildet ist, und einen zweiten Einklemmspalt enthält, der in dem zweiten Schlaufensegment ausgebildet ist.

22. An einer Iris zu fixierende intraokulare Linse nach Anspruch 14, bei der der Einklemmspalt eine Breite von zwischen ungefähr 0,05 mm und ungefähr 0,25 mm und eine Länge von zwischen ungefähr 0,2 mm und ungefähr 0,5 mm hat.

23. An einer Iris zu fixierende intraokulare Linse nach Anspruch 1, bei der die Linse eine dreiteilige Linse ist,
wobei die Optik aus einem elastisch verformbaren Kunststoffmaterial ausgebildet ist,
wobei die Befestigungselemente ein erstes und ein zweites Befestigungselement umfassen, und
wobei der flexible Strang einen einzelnen flexiblen Strang aufweist.

24. Dreiteilige, an einer Iris zu fixierende intraokulare Linse nach Anspruch 23, bei der das erste und das zweite Befestigungselement im Wesentlichen identisch zueinander sind und an entgegengesetzten Seiten der optischen Achse an der Optik befestigt sind.

25. Dreiteilige, an einer Iris zu fixierende intraokulare Linse nach Anspruch 23, bei der der mindestens eine Einklemmspalt in der am distalen Ende angeordneten Schlaufe von jedem des ersten und des zweiten Befestigungselements in einem Bereich der Schlaufe angeordnet ist, der sich am dichtesten an der optischen Achse befindet.

26. An einer Iris zu fixierende intraokulare Linse nach Anspruch 23, bei der der mindestens eine Einklemmspalt in der am distalen Ende angeordneten Schlaufe von jedem des ersten und des zweiten Befestigungselements in einem Bereich der Schlaufe angeordnet ist, der am weitesten von der optischen Achse entfernt ist.

## Revendications

1. Lentille intraoculaire fixée à l'iris (20) qui comprend :
(a) une optique (50) ayant un axe optique et des côtés antérieur et postérieur (60, 62) ; et
(b) au moins deux éléments de fixation (52), chacun desdits éléments de fixation ayant une région d'extrémité proximale (70) et une région d'extrémité distale (72), ladite région d'extrémité proximale comprenant un brin flexible fixé à une région de bordure de ladite optique de façon à s'étendre généralement tangentiellement vers l'extérieur à partir de celle-ci, et ladite région d'extrémité distale étant formée suivant une boucle, **caractérisée par le fait que** dans ladite boucle est défini au moins un intervalle étroit (78) de pincement d'iris qui a une largeur sensiblement uniforme et est dimensionnée pour pincer un petit segment de surface de tissu d'iris dans ledit intervalle pour attacher de façon détachable ladite lentille intraoculaire à une surface antérieure d'iris.

2. Lentille intraoculaire fixée à l'iris, selon la revendication 1, dans laquelle ledit au moins un intervalle de pincement est situé sur une ligne généralement perpendiculaire audit axe optique et est positionné à un rayon à partir dudit axe optique d'environ 4,25 mm.

3. Lentille intraoculaire fixée à l'iris, selon la revendication 2, dans laquelle lesdits premier et second éléments de fixation sont construits séparément de ladite optique, ladite lentille intraoculaire étant par là une lentille intraoculaire en trois pièces.

4. Lentille intraoculaire fixée à l'iris, selon la revendication 3, dans laquelle ladite optique est construite à partir d'une matière élastiquement déformable.

5. Lentille intraoculaire fixée à l'iris, selon la revendication 4, dans laquelle ladite matière élastiquement déformable est une matière de silicone ou une matière acrylique.

6. Lentille intraoculaire fixée à l'iris, selon la revendication 1, dans laquelle les boucles d'extrémités distale de chacun desdits au moins deux éléments de fixation s'étendent dans au moins un plan sensiblement commun situé à l'arrière de ladite optique.

7. Lentille intraoculaire fixée à l'iris, selon la revendication 1, dans laquelle ladite boucle d'extrémité distale de chacun desdits au moins deux éléments de fixation est allongée en une forme incurvée.

8. Lentille intraoculaire fixée à l'iris, selon la revendication 1, dans laquelle :
- la boucle d'extrémité distale de chacun desdits au moins deux éléments de fixation est allongée en une forme incurvée, chacune desdites boucles d'extrémité distale comprenant un moyen divisant lesdites boucles en un premier et un second segment.

9. Lentille intraoculaire fixée à l'iris, selon la revendication 8, dans laquelle ledit élément de division s'étend généralement perpendiculairement audit axe optique de ladite optique.

10. Lentille intraoculaire fixée à l'iris, selon la revendication 8, comprenant un premier intervalle de pincement défini dans ledit premier segment de boucle et un second intervalle de pincement défini dans ledit second segment de boucle.

11. Lentille intraoculaire fixée à l'iris, selon la revendication 1, dans laquelle ledit intervalle de pincement est défini à un angle par rapport à ladite ligne perpendiculaire.

12. Lentille intraoculaire fixée à l'iris, selon la revendication 1, dans laquelle ledit au moins un intervalle de pincement a une largeur d'entre environ 0,05 mm et environ 0,25 mm, ou dans laquelle ledit au moins un intervalle de pincement a une longueur d'entre environ 0,2 mm et environ 0,5 mm.

13. Lentille intraoculaire fixée à l'iris, selon la revendication 1, dans laquelle la lentille intraoculaire a un diamètre global d'entre environ 7,5 mm et environ 10 mm.

14. Lentille intraoculaire fixée à l'iris, selon la revendication 1, dans laquelle ledit brin flexible est un brin flexible unique.

15. Lentille intraoculaire fixée à l'iris selon la revendication 14, dans laquelle lesdits premier et second éléments de fixation sont sensiblement identiques l'un à l'autre et sont attachés à ladite optique sur des côtés opposés dudit axe optique.

16. Lentille intraoculaire fixée à l'iris, selon la revendication 14, dans laquelle les boucles d'extrémité distale de chacun desdits premier et second éléments de fixation s'étendent dans un plan généralement commun, et dans laquelle lesdits intervalles de pincement sont situés sur un diamètre, D, à travers l'axe optique de ladite optique.

17. Lentille intraoculaire fixée à l'iris, selon la revendication 16, dans laquelle ladite distance, D, est entre environ 8,0 mm et environ 9,0 mm.

18. Lentille intraoculaire fixée à l'iris, selon la revendication 14, dans laquelle ladite boucle d'extrémité distale de chacun desdits premier et second éléments de fixation est allongée en une forme incurvée ayant un grand axe généralement parallèle audit axe optique.

19. Lentille intraoculaire fixée à l'iris, selon la revendication 14, dans laquelle l'intervalle de pincement dans la boucle d'extrémité distale de chacun desdits premier et second éléments de fixation est situé dans une région de ladite boucle la plus proche dudit axe optique.

20. Lentille intraoculaire fixée à l'iris, selon la revendication 1,
- dans laquelle lesdits éléments de fixation comprennent des premier et second éléments de fixation ;
- dans laquelle ledit brin flexible est un brin flexible unique et dans laquelle ledit intervalle de pincement d'iris étroit est situé sur une ligne généralement perpendiculaire audit axe optique, l'intervalle de pincement dans la boucle d'extrémité distale de chacun desdits premier et second éléments de fixation étant situé dans une région de ladite boucle la plus éloignée dudit axe optique.

21. Lentille intraoculaire fixée à l'iris, selon la revendication 1, dans laquelle : lesdits éléments de fixation comprennent des premier et second éléments de fixation ; ledit brin flexible est un brin flexible unique ; et dans laquelle ledit intervalle étroit de pincement d'iris est situé sur une ligne généralement perpendiculaire audit axe optique, chacune desdites boucles d'extrémité distale comprenant un moyen divisant la boucle en un premier et un second segment de boucle, ledit moyen de division s'étendant le long d'une ligne généralement perpendiculaire audit axe optique, et comprenant un premier intervalle de pincement défini dans ledit premier segment de boucle et un second intervalle de pincement défini dans ledit second segment de boucle.

22. Lentille intraoculaire fixée à l'iris, selon la revendication 14, dans laquelle ledit intervalle de pincement a une largeur d'entre environ 0,05 mm et environ 0,25 mm et une longueur d'entre environ 0,2 mm et environ 0,5 mm.

23. Lentille intraoculaire fixée à l'iris selon la revendication 1,
- dans laquelle la lentille est une lentille en trois pièces ;
- dans laquelle ladite optique est construite à partir d'une matière plastique élastiquement déformable ;
- dans laquelle lesdits éléments de fixation comprennent un premier et un second élément de fixation ; et
- dans laquelle ledit brin flexible comprend un brin flexible unique.

24. Lentille intraoculaire fixée à l'iris en trois pièces selon la revendication 23, dans laquelle lesdits premier et second éléments de fixation sont sensiblement identiques l'un à l'autre et sont attachés à ladite optique sur des côtés opposés dudit axe optique.

25. Lentille intraoculaire fixée à l'iris en trois pièces selon la revendication 23, dans laquelle ledit au moins un intervalle de pincement dans la boucle d'extrémité distale de chacun desdits premier et second éléments de fixation est situé dans une région de ladite boucle la plus proche dudit axe optique.

26. Lentille intraoculaire fixée à l'iris selon la revendication 23, dans laquelle ledit au moins un intervalle de pincement dans la boucle d'extrémité distale de chacun desdits premier et second éléments de fixation est situé dans une région de ladite boucle la plus éloignée dudit axe optique.
